# EUROPEAN PATENT APPLICATION

(11) **EP 4 176 718 A1**
(43) Date of publication of application: **10.05.2023**
(21) Application number: 22205291.2
(22) Date of filing: 03.11.2022
(51) Int. Cl.: A01M 1/20, A61L 9/12

(54) **EVAPORATOR DEVICE WITH AIR FRESHENER AND BIOCIDE**

(30) Priority: 03.11.2021 ES 202132158 U
(71) Applicant: ZENIT Estudio de Diseño e Innovación S.L., 46010 Valencia (ES)
(72) Inventor: Blasco Feo, Vicente, Valencia (ES)
(74) Representative: Soler Lerma, Santiago

(57) **Abstract**

Device for dispersing substances comprising a ventilated box and, therein, an air freshener element and a biocidal element, both elements being independent of each other, without contact between them due to the action of a separator, and each with a specific ventilation. This ventilated box comprises a lid and a rear housing that are attached. The lid has at least two independent ventilation areas, one that acts to a greater extent with the biocide and another that acts to a greater extent with the air freshener.

## Description

As its name indicates, the invention relates to a device with an air freshener element and a biocidal element both contained in a housing that houses them without coming into contact with one other and where the housing allows independent ventilation of both the air freshener element and the biocidal element.

The field of action of this invention is that of volatile substance evaporators.

### BACKGROUND

Currently, there is a wide variety of air fresheners with insecticide component, such as those aimed at having an insecticidal effect inside closed rooms such as spaces intended to house all kinds of fabrics, clothing, etc.

Utility model ES0041767 is a clear example of these types of products since it comprises a device consisting of a cylindrical block with a concentric orifice made on its vertical axis, composed of insecticide, disinfectant and perfuming material, where the central orifice performs the function of allowing the block to be hung either by means of a loop attached to a hanger or arranged on the hook of said hanger. This type of embodiment raises the manufacturing costs and complicates the device as a whole.

Another example can be seen in utility model ES0143302 where the invention consists of an anti-moth deodorant block wrapped in cellophane paper where said block has an interior space to house part of a flexible wire where the other end performs the function of a hook protruding from it.

Or utility model ES0158289 which consists of a device for suspending tablets, preferably anti-moth in order to cover a greater space to be protected. The device can have a convenient number of tablets on its structure where its upper end has a hook that enables it to be anchored to any surface that allows it.

These types of arrangements can have the disadvantage of coming into direct contact with the surrounding elements, which can cause stains due to the transfer of unwanted colours or odours or also that the block or the tablet can be fractured or break into pieces.

The use of housings to contain such tablets solves said problems, with the aim that the tablets do not come into direct contact with fabrics, clothing, etc.

From the literature that can be gathered on devices to house said tablets, we have utility model ES0155450, which relates to a support for volatile tablets with a hook to allow a hanging placement where said support is a flexible strip bent over itself to enable a housing between the two faces present since they are bent.

Likewise, utility model ES0158603 consists of a tubular-shaped receptacle with a closed bottom and with a closure that has a series of windows facing each other diametrically that allow the penetration of the air oxidising the solid and the latter to give off the corresponding emanations or discharges.

Normally the tablets or volatile solids contained within these devices include a biocide in their composition that complements the insecticidal effect together with the air freshener of the product where usually, when the perfuming effect of the tablets ends, it is thrown in the bin entailing an environmental problem since the contained biocide element usually continues to emanate its discharge into the environment which can affect the surrounding fauna.

This type of device implies that the tablet or solid disposed inside the container does not give the option of being able to introduce at least two types of evaporating elements separately for different purposes, whether insecticides or air fresheners, and not providing independent ventilation for each evaporating element.

### DESCRIPTION OF THE INVENTION

To solve the problems stated, the invention relates to a device comprising a ventilated box, suitable for housing therein an element with an air freshener substance which we will call an air freshener and an element with a biocidal substance which we will call a biocide, both elements being independent of each other, without contact between one another, and each with a specific ventilation.

The device comprises a lid and a rear housing with means for joining to form a box suitable for housing the air freshener and the biocide.

In a possible embodiment, the device comprises a hook by way of a hanger to facilitate its hanging use.

In a preferred embodiment, the device, on the inside of the lid, has two partitions, preferably concentric, one perimeter and the other inner that define a circular passage between them.

In a possible embodiment, both the outer and inner partitions have projections oriented towards the passage existing between the partitions, these projections being suitable for retaining the biocidal element.

The lid has at least two independent ventilation areas, preferably a central area for the air freshener element and a perimeter area for the biocide element.

The arrangement of the partitions, especially the interior, allows discriminating between the central ventilation area, which we will call the chimney, and the perimeter ventilation area.

The arrangement and size of the windows in each of these areas provides each of the elements, air freshener or biocide, with adequate ventilation to control that its wear is simultaneously reaching the end of its useful life.

The biocidal substance is impregnated on a support whose geometry, preferably, adapts to the interior geometry of the lid, in particular to the passage defined between both partitions, perimeter and interior, venting through the perimeter ventilation area.

The air freshener comprises a support and a fragrance and is arranged in the housing in a suitable hole venting through the chimney. Preferably, the support is of carrageenan and has a thick disc shape of suitable size and shape to house in the hollow of the housing.

The biocide comprises a support and a biocidal substance that impregnates it. For this purpose, the support is made in a porous substance such as cellulose membrane. The support has adequate dimensions and shape to house on the inside of the lid, in the passage between the perimeter and inside partitions, and be fastened by the projections existing thereon in such a way that one of its faces is facing the perimeter area of air freshener and the other the biocide element. In a preferred embodiment, the support is O-shaped.

The biocide element has, on its face facing the air freshener, a separator, such as a film, which prevents its contact with the air freshener and prevents the biocide from evaporating through its lower part by the action of the ventilation of the chimney, all to control that they are consumed in the planned simultaneous way.

### DESCRIPTION OF THE FIGURES

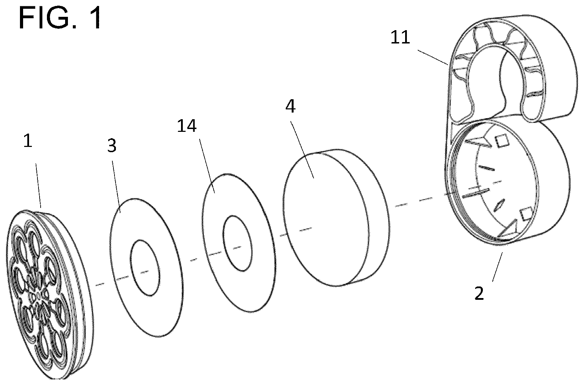

Fig. 1 A preferred embodiment of the device is shown in exploded form where the lid (1) and the rear housing (2) with their extension in the form of a hanger (11), the biocide (3) and the air freshener (4) can be observed.

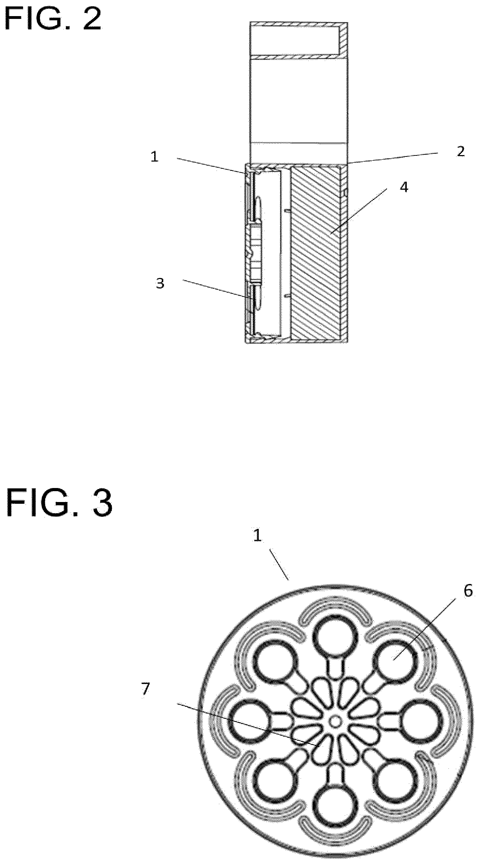

Fig. 2 shows in a preferred embodiment a side section that displays the lid (1) coupled to the housing (2), the biocidal product (3) and the air freshener (4) which limit inside the device without coming into direct contact with one another thanks to a separator (14) that prevents direct contact between the biocidal product (3) and the air freshener (4).
Fig. 3 shows one of the possible embodiments of the shape of the lid (1) where two ventilation areas are seen, the perimeter ventilation area (6) on the outermost part of the lid (1) which communicates the biocide with the outside and the outer part of the chimney (7) on the inside of the lid (1) which communicates the air freshener with the outside of the device.

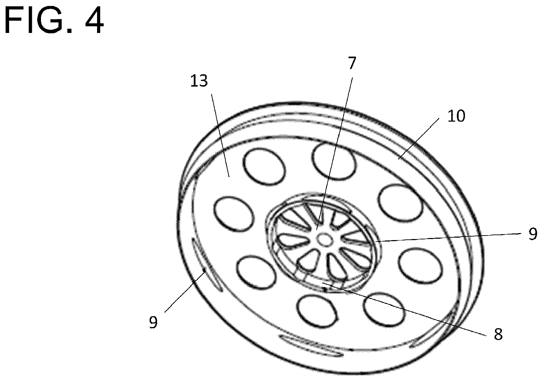

Fig. 4 shows a perspective view of the possible embodiment of the lid (1) where the interior part of the lid is observed comprising an outer partition (10) that collaborates in fixing the lid to the housing, an inner partition (8) that defines the chimney (7), a passage (13) formed between both partitions and projections (9) towards that passage (13) suitable to retain the biocide.

### DESCRIPTION OF AN EMBODIMENT

The following describes an embodiment of the invention that is not limiting but merely explanatory.

The invention relates to a device combining air freshening and biocidal effect wherein the emission of the substances into the atmosphere is produced by evaporation by action of air coming into contact with the biocidal product and with the air freshener. To this end, the device comprises:
A ventilated box comprising a rear housing (2) and a lid (1) that closes it.
- The lid comprises a series of perforations or windows distributed in a perimeter ventilation area (6) and a chimney (7) or internal ventilation area and, on the inner face of the lid, an outer partition (10) running along the perimeter and an inner partition (8) both concentric, a passage (13) defined between both partitions and a chimney (7) enclosed by the inner partition.
- The housing (2) has a container shape with an inner hollow and a hanger-shaped extension (11).
- Attached to the lid by its lower part, an O-shaped biocide (3) comprising an impregnable support, a biocidal substance impregnated in said support and a separator (14).
- Housed in the housing (2), an air freshener (4) in the form of a thick disc comprising a support, such as a carrageenan body, and a fragrance.

The device also has a hanger-shaped extension (11) that enables it to be used inside cabinets or wardrobes and, in general, in any place where there is a bar or projection whereon it can be hung.

The lid has two independent ventilation areas, a perimeter one for the biocide (3) and the chimney for the perfumed carrageenan air freshener (4).

The coupling of the lid (1) with the rear housing (2) is generated by pressure and complementary geometries, with the outer partition (8) being inserted into the rear housing (2).

As has been mentioned, the lid has two partitions in its lower part that are the outer partition (10) that runs along the maximum perimeter of the lid (1) and, the inner partition (8) with a smaller perimeter, both being concentric.

The height of the outer partition (10) is greater than the height of the inner partition (8).

Both partitions have projections (9) oriented towards the passage (13) that retain the biocidal product (3) with some clearance.

The perfumed carrageenan air freshener (4) has a disc shape with a geometry and measurements suitable to house in the housing, and the biocide has O-shaped geometry and dimensions suitable to house in the passage (13) and to be fastened in the projections (9) of the partitions.

The external ventilation area (6) enables the outlet of the discharge of the biocide (3) and the chimney favours the ventilation of the air freshener.

A separator (14) is arranged between the biocidal product (3) and the air freshener (4), in this case an adhered film, which prevents contact between the biocidal product and the air freshener.

The outer face of the biocide (3) is facing the perimeter area (6).

## Claims

1. EVAPORATING DEVICE WITH AIR FRESHENER and BIOCIDE comprising a cap (1) and a rear housing (2) that are joined forming a box **characterised in that** therein are housed a biocide (3), an air freshener (4) and a separator arranged between them and **in that** it comprises a perimeter area (6) in the perforated cap (1) of adequate ventilation for evaporating the biocide and a chimney (7) adequate for evaporating the air freshener.

2. EVAPORATING DEVICE WITH AIR FRESHENER AND BIOCIDE according to claim 1, **characterised in that** the lid has on its inner face a perimeter partition (10), an inner partition (8) and a passage (13) between them.

3. EVAPORATING DEVICE WITH AIR FRESHENER AND BIOCIDE according to claim 2, **characterised in that** the perimeter passage (10) is higher than the inner partition (8).

4. EVAPORATING DEVICE WITH AIR FRESHENER AND BIOCIDE according to claim 2, **characterised in that** the partitions (8) and (10) have projections (9) facing the passage (13).

5. EVAPORATING DEVICE WITH AIR FRESHENER AND BIOCIDE according to claim 2, **characterised in that** the closure of the lid and the housing is produced by geometry and pressure, the perimeter partition being inserted into the housing.

6. EVAPORATING DEVICE WITH AIR FRESHENER AND BIOCIDE according to claim 4, **characterised in that** the biocide is O-shaped of suitable dimensions to be housed in the passage (13) and fastened in the projections (9).

7. EVAPORATOR DEVICE WITH AIR FRESHENER AND BIOCIDE according to claim 1, **characterised in that** the air freshener (4) is in the form of a thick disc and is housed in the housing.
